# EUROPEAN PATENT APPLICATION

(11) **EP 1 688 085 A1**
(43) Date of publication of application: **09.08.2006**
(21) Application number: 05002074.2
(22) Date of filing: 02.02.2005
(51) Int. Cl.: A61B 5/00, A61M 5/142

(54) **Ambulatory medical device and method of communication between medical devices**

(71) Applicant: Disetronic Licensing AG, 3401 Burgdorf (CH)
(72) Inventor: Essenpreis, Matthias, 3400 Burgdorf (CH); Haueter, Ulrich, 3506 Grosshöchstetten (CH); Bernini, Nicole, 3423 Ersigen (CH); Fankhauser, Sybille, 3052 Zollikofen (CH); La Bastide Sebastiaan, 3074 Muri b.Bern (CH); Meyer Olden, Gunnar, 3400 Burgdorf (CH); Schoemaker, Michael, 68183 Mannheim (DE); Heaton, Kelly, 3423 Ersigen (CH)
(74) Representative: Küng, Peter

(57) **Abstract**

The present invention relates to an ambulatory medical device and a method of communication between medical devices. The inventive medical device comprises a module for communication with at least a second medical device wherein said module for communication is adapted to be activated by a value of a physiological parameter of an animal. The method of the present invention comprises a first medical device and at least a second medical device wherein the communication between said medical devices is activated by a value of a physiological parameter of an animal.

## Description

### Technical field of the invention

The present invention relates to an ambulatory medical device and a method of communication for medical devices.

### Background Art

Ambulatory medical devices for the treatment of diabetes include e.g. extra corporal insulin pumps and blood glucose measuring devices such as e.g. hand held glucose meters. Insulin pumps allow a good control of blood glucose concentrations by continuously infusing a basic amount of insulin in a human body (basal insulin rate) and manually controlled additional "meal bolus" insulin quantities thereby reflecting the insulin secretion by the pancreas. Furthermore, the development of continuous glucose sensors will allow to measure in vivo glucose concentrations over the whole day. The measured glucose date can be used to adjust the diabetes therapy to individual needs.

In order to improve the treatment of diabetes it is important to provide means and methods to transfer data between medical devices in a quality assuring way

It is therefore the aim of the present invention to provide a medical device allowing a controlled data transfer between medical devices and a method of controlled data transfer.

### Disclosure of the invention

In a first aspect, the present invention relates to a medical device comprising a module for communication with at least a second medical device wherein the module for communication in said medical device is adapted to be activated by a value of a physiological parameter of an animal.

The inventive medical device preferably comprises a telemetry system for wireless communication, preferably a telemetry system for RF communication.

Preferably the medical device is selected from the group consisting of a remote control, a PDA, an analyte measuring device, preferably a glucose measuring device such as e.g. a hand held glucose meter, more preferably a strip based glucose meter or combinations thereof.

The physiological parameter is preferably selected from the group consisting of an analyte concentration, a physiological characteristic like conductivity of an animal, a physiological vital sign like heart or breath rate, temperature, movement, air- or structure-bome sound, ECG (electrocardiogram) and the like.

In a preferred embodiment of the present invention the analyte concentration is a blood glucose concentration.

Preferably, the medical device of the present invention comprises an electrochemical or photometric module for measuring blood glucose. Suitable medical devices are e.g. strip based glucose meters such as AccuChek Compact.

In a second aspect the present invention relates to a system of medical devices. Said system comprises a first medical device of the present invention as described in the previous section and at least a second medical device capable to communicate with said first medical device.

In a preferred embodiment the second medical device is selected from the group consisting of an extra corporal infusion pump, an implantable infusion pump, a pacesetter, an analyte or vital sign sensor, preferably a continuous analyte or vital sign sensor, more preferably a continuous glucose sensor.

In a further preferred embodiment the first medical device and the at least second medical device comprise a telemetry system for wireless communication, preferably a telemetry system for RF communication.

In a third aspect, the present invention relates to a method of communication between a first medical device and at least a second medical device wherein the communication between said medical devices is enabled and/or activated by a value of a physiological parameter of an animal.
In a preferred embodiment, the communication between the at least two medical devices is enabled and/or activated for a predetermined time. Time duration can be fixed, random or dependent on the physiological parameter enabling and/or activating the communication or other physiological parameters of the animal body.

The physiological parameter is preferably selected from the group consisting of an analyte concentration, a physiological characteristic like conductivity of an animal, a physiological vital sign like heart or breath rate, temperature, movement, air- or structure-bome sound, ECG (electrocardiogram) and the like. Preferably the analyte concentration is a blood glucose concentration.

In a preferred embodiment, the activation of communication between the medical devices is performed on the first medical device by a value of the physiological parameter.

In a further preferred embodiment, the first medical device is selected from the group consisting of a remote control, a PDA, an analyte measuring device, preferably a glucose measuring device, more preferably a strip based glucose meter.

The second medical device is preferably selected from the group consisting of an extra corporal infusion pump, an implantable infusion pump, a pacesetter, an analyte sensor, preferably a continuous analyte sensor, more preferably a continuous glucose sensor.

In a preferred embodiment, the communication between said medical devices is a wireless communication, preferably a RF communication.

In a further preferred embodiment, the first medical device receives data from the second medical device.

In a further preferred embodiment, the first medical device sends commands to the second medical device controlling at least partially the function of said device.

### Detailed description of the invention

In one aspect, the present invention relates to a novel method for controlling and/or enabling communication between medical devices, in particular medical sensory devices such as continuous glucose sensors and/or therapeutic devices such as insulin pumps and/or diagnostic medical devices such as glucose meters.

For example, the communication between a continuous glucose sensor applied to a human body and a blood glucose meter can only be established when a blood glucose measurement has been made in the blood glucose meter. The generation of the blood glucose value in the blood glucose meter enables and/or activates communication between the two devices for a specified time limit. During the time window data can be transferred from the sensor to the glucose meter and/or commands from the glucose meter can be sent to the sensor. After expiration of a time limit, communication between the two devices is deactivated. In order to establish a further communication, the communication link between the two devices has to be activated by generating a further blood glucose value in the glucose meter.

The term "generation of a value" as it is used herein encompasses any method or procedure for the determination of physiological parameters such as methods for the measurement of analyte values, in particular blood glucose values. Suitable methods for the determination of blood glucose values are e.g. electrochemical methods and photometric methods which are known to a person skilled in the art.

The dependence of the communication link between medical devices on an actual analyte value ensures the quality of the data transmitted from the medical sensory device and/or medical therapeutical device to the medical diagnostic device.

The data transferred from the sensor to the diagnostic device can be stored on the diagnostic device e.g. a glucose meter and be transferred to a third device such as a PDA or a computer, for further processing and/or analysis. By means of suitable software the data can be analyzed and used for e.g. bolus recommendation or adjustment of basal insulin rates for patients using an extra- or intra corporal insulin pump.

The communication link between the diagnostic device and a third device e.g. a computer, does not need activation by generation of a blood glucose value in said diagnostic device.

In a preferred embodiment, the present invention relates to a method of communication between a diagnostic medical device, preferably a blood glucose meter, and an infusion pump, preferably an extra corporal insulin pump. In this specific embodiment, the diagnostic medical device is used as a remote control to control the function of the infusion pump. After a blood glucose value has been generated in the blood glucose meter a communication link between meter and pump is enabled and/or activated for a defined time and commands can be transferred from the remote control i.e. the glucose meter, to the pump. It is as well possible to transfer data stored on the pump to the diagnostic device during the communication time window.

When the remote control of the infusion pump does not comprise a module for measuring blood glucose concentration, the communication between pump and remote control is activated by entering a current blood glucose value measured in a blood glucose meter in the remote control. The value can e.g. be entered using buttons of the remote control or can be transferred via a wireless or wired connection to the glucose meter. After the blood glucose value has been entered in the remote control, a communication link between remote control and infusion pump is established, preferably for a predetermined time span. After expiration of the time span, the communication is interrupted and no data exchange between the two devices is anymore possible. A further round of communication needs a new activation of the communication by entering a new, current blood glucose value in the remote control. The term remote control as used herein encompasses PDA, smart phones, a handy and pump specific remote controls. The data transfer between the medical devices can be performed using known technologies and comprise wired connections as well as wireless communications. These technologies are known to a person skilled in the art. The preferred communication is a wireless communication, more preferably RF communication.

The data transfer between the devices can be encrypted in order to ensure that non-authorized third parties do not gain access to personal data of patients. Methods of encrypting data are known to a person skilled in the art.

In a further preferred embodiment, the communication between the medical devices is activated by a manipulation on the second medical device e.g. an insulin pump, such as pressing a button or lever, inserting a battery, using the touch screen, shaking, bumping or squeezing or the like.

In the following paragraph a preferred embodiment of the present invention is described.

The preferred system of medical devices comprises a continuous glucose sensor device which is placed on a human body in order to measure glucose value in interstitial fluid and a glucose meter. The sensor device comprises an electrochemical glucose sensor measuring the glucose concentration in the interstitial tissue in a predetermined manner. The sensor device further comprises an extra corporal part including processor means for controlling the sensor, memory for storing measured glucose values and a telemetry system for transmitting the data to a glucose meter, preferably a strip-based glucose meter. The glucose values stored on the sensor device are then transferred to a glucose meter via the telemetry system.

The communication between the two devices i.e. the wireless link, is established and/or activated by measuring the glucose concentration in a blood sample of a patient using the glucose meter, preferably a strip-based glucose meter. When a strip-based glucose meter is used, the patient inserts a strip in the glucose meter and puts a droplet of blood on the strip. The glucose meter measures and indicates on its display the blood glucose value. After measurement of the blood glucose value, the communication link can then be activated/established either by pressing a button on the glucose meter e.g. an activation button or by a direct electronic link to the processor controlling the glucose telemetry system such that the completion of the blood glucose measurement automatically activates the wireless link between the devices.

The communication link is then established and a data transfer between the medical devices is possible for a defined time span. After expiration of the defined time span the communication link is deactivated and no further data/commands can be transmitted between the medical devices. A new blood glucose measurement in the glucose meter is then necessary to open a new wireless link between the medical devices.

In a further aspect, the present invention relates to a method of data processing or data use. Said method is characterised in that data processing or data use is only possible after activation by a value of a physiological parameter. The method is preferably used for the processing of medical data such as data measured by a sensor applied on a human body.

In a preferred embodiment, the method is used for the processing of medical data which have been measured by a sensor device applied on a human being, preferably a continuous glucose sensor. The data are then e.g. transferred to a diagnostic medical device, preferably a blood glucose meter. The data are preferably transferred via a wireless link from the sensor device to the diagnostic device. In this case, each of the at least two medical devices comprises a telemetry system for wireless communication. The wireless communication can be bidirectional or unidirectional.

There is preferably a permanent communication link between said two medical devices but the data stored e.g. in the memory of the medical sensor device and transferred to the diagnostic device can only be further processed on the diagnostic device after the processing has been activated by a value of a physiological parameter. After activation by a value of a physiological parameter, preferably a blood glucose value, the data stored in the memory of the diagnostic device can be processed or used. For example, data are transferred from a continuous glucose sensor to a glucose meter and stored in the memory of the glucose meter. The further processing of these data is then only possible after activation of the processing by a value of a physiological parameter, preferably a blood glucose value.

In a preferred embodiment, the processing of the data is only possible for a limited time span after activation by a value of a physiological parameter. When the defined time span for data processing has lapsed, no further data processing is possible without a new activation by a value of a physiological parameter.

In a further aspect, the present invention relates to a medical device comprising a module for data processing which is adapted to be activated by a value of a physiological parameter. Preferably said module comprises a microprocessor with a memory for storing data.

Said medical device is preferably a blood glucose meter. Preferably, the processing of data stored in the memory of the blood glucose meter is either activated by pressing a button on the glucose meter (an activation button) or by a direct electronic link to the processor controlling the data processing module/system such that the completion of the blood glucose measurement automatically activates data processing

The terms "data processing" or "data use" as they are used herein refer to any manipulation of data and comprise analysis of data, presentation of data, interpretation of data and indication of data.

## Claims

1. A medical device comprising a module for communication with at least a second medical device wherein the module for communication in said first medical device is adapted to be activated by a value of a physiological parameter of an animal.

2. The medical device of claim 1, wherein the module for communication is a telemetry system for wireless communication, preferably a telemetry system for RF communication.

3. The medical device of claim 1 or 2, wherein the medical device is selected from the group consisting of a remote control, a PDA, an analyte measuring device, preferably a glucose measuring device, more preferably a strip based glucose measuring device.

4. The medical device of claims 1 to 3, wherein the physiological parameter is an analyte concentration of an animal.

5. The medical device of claim 4, wherein the analyte concentration is a blood glucose concentration.

6. The medical device of claim 5, wherein the first medical device comprises an electrochemical module for measuring blood glucose.

7. The medical device of claim 5, wherein the first medical device comprises a photometric module for measuring blood glucose.

8. A system of medical devices comprising:
a) a first medical device according to claims 1 to 7 and
b) at least a second medical device capable to communicate with said first medical device.

9. The system of claim 8, wherein the second medical device is selected from the group consisting of an extra corporal infusion pump, an implantable infusion pump, a pacesetter, an analyte sensor, preferably a continuous analyte sensor, more preferably a continuous glucose sensor.

10. The system of claim 8 or 9, wherein the first medical device and the at least second medical device comprise a telemetry system for wireless communication, preferably a telemetry system for RF communication.

11. A method of communication between a first medical device and at least a second medical device wherein the communication between said medical devices is enabled and/or activated by a value of a physiological parameter of an animal.

12. The method of claim 11, wherein the communication is enabled and/or activated for a predetermined time window.

13. The method of claim 12, wherein the time window is established according to a value of a physiological characteristic of an animal or according to a set of data gained during previous time periods.

14. The method of claims 11 to 13, wherein the physiological parameter is selected from the group consisting of an analyte concentration of an animal, a heart rate, an electrophysiological value, preferably a blood glucose concentration.

15. The method of claim 11 to 14, wherein the activation of the communication is performed on the first medical device by generating a value of the physiological parameter in said first medical device.

16. The method of claims 11-15, wherein the first medical device is selected from the group consisting of a remote control, a PDA, an analyte measuring device, preferably a glucose measuring device, more preferably a strip-based glucose measuring device.

17. The method of claims 11-16, wherein the second medical device is selected from the group consisting of an extra corporal infusion pump, an implantable infusion pump, a pacesetter, an analyte sensor, preferably a continuous analyte sensor, more preferably a continuous glucose sensor.

18. The method of claims 11-17, wherein the communication between said medical devices is a wireless communication, preferably a RF communication.

19. The method of claims 11-18, wherein the first medical device receives data from the second medical device.

20. The method of claims 11-19, wherein the first medical device sends commands to the second medical device to control at least partially said second device.
